# EUROPÄISCHE PATENTSCHRIFT

(11) **EP 0 404 781 B1**
(45) Veröffentlichungstag und Bekanntmachung des Hinweises auf die Patenterteilung: **24.11.1993**
(21) Anmeldenummer: 89902519.1
(22) Anmeldetag: 24.02.1989
(51) Int. Cl.: A61B 5/00

(54) **VERFAHREN UND VORRICHTUNG ZUR MESSUNG DER ABSORPTION VON STRAHLUNG IN EINEM GEWEBE**
PROCESS AND DEVICE FOR MEASURING THE RADIATION ABSORBED BY A TISSUE
PROCEDE ET DISPOSITIF POUR MESURER L'ABSORPTION DE RADIATIONS PAR UN TISSU

(30) Priorität: 24.03.1988 DE 3810008
(43) Veröffentlichungstag der Anmeldung: 02.01.1991
(73) Patentinhaber: BUSCHMANN, Johannes, D-81543 München (DE)
(72) Erfinder: BUSCHMANN, Johannes, D-81543 München (DE)
(86) Internationale Anmeldenummer: EP8900170
(87) Internationale Veröffentlichungsnummer: WO8909016

(56) Entgegenhaltungen:
- EP-A- 0 104 619
- EP-A- 0 135 840

## Beschreibung

Die Erfindung betrifft ein Verfahren und eine Vorrichtung zur Messung der Absorption von Strahlung in einem Gewebe nach dem Oberbegriff des Auspruchs 1 oder 2 unter Verwendung einer Sensoreinrichtung.

Bei der Überwachung physiologischer oder biochemischer Größen biologischen Gewebes ist es wünschenswert, im Rahmen einer laufenden Meßwertaufnahme die zu ermittelnden Daten jeweils direkt und mit hoher Präzision ermitteln zu können.

So ist es bei der Vorbereitung und/oder Überwachung der Geburtsphase eines Kindes beispielsweise wünschenswert, den Gesundheitszustand des Feten laufend zu überwachen und Meßwerte zur Hand zu haben, welche Aussagen über den aktuellen Zustand des Kindes, insbesondere die Sauerstoffversorgung des Feten, ermöglichen.

Es ist bisher üblich, zur Überwachung des Feten seine Herzfrequenz kontinuierlich zu bestimmen, entweder über ein fetales EKG oder über die Registrierung herzschlagsynchroner Bewegungen mit Hilfe von Ultraschall. Veränderungen der fetalen Herzfrequenz sind jedoch, auch wenn sie mit der mütterlichen Wehentätigkeit korreliert betrachtet werden, nicht sehr spezifisch hinsichtlich des fetalen Wohlergehens.

Ferner ist aus dem Stand der Technik die kontinuierliche Messung des fetalen O₂- und CO₂ Partialdruckes bekannt. Diese Meßparameter wären zwar von hohem klinischen Interesse, jedoch haben die verwendeten Meßverfahren gravierende theoretische und praktische Nachteile, die eine routinemäßige Verwendung nicht ermöglichen: Abgesehen davon, daß derartige transkutante Messungen vielfach nicht die erforderliche Genauigkeit und Reproduzierbarkeit aufweisen, erweist es sich als nachteilig, daß die Sonden einen relativ großen Durchmesser haben und damit erst applizierbar sind, wenn der Muttermund schon relativ weit geöffnet ist. Ein weiterer Nachteil ist, daß die Sonden unter Sicht an der fetalen Haut befestigt werden müssen und somit für das Anbringen und die Kontrolle eine "vaginale Einstellung" nötig wird. Ebenfalls nachteilig ist, daß die Haut unter der Sonde bei diesen Meßverfahren evtl.zur Erzeugung einer Hyperämie relativ stark erwärmt werden muß. Bei längeren Meßperioden können durch die Erwärmung Hautschäden auftreten.

Aus dem Stand der Technik ist es weiterhin bekannt, am menschlichen Körper eine Messung der Sauerstoffsättigung des Blutes auf optische Weise transkutan dadurch vorzunehmen, daß ein Gewebebereich durchleuchtet wird, wobei durch Auswahl geeigneter Strahlungswellenlängen der Anteil des O2-beladenen Hämoglobins bestimmbar ist. Derartige Messungen erfordern eine Meßvorrichtung, welche einen Körperteil umgreift, um diesen zu durchleuchten. Aus dem Stand der Technik ist es bekannt, derartige Meßvorrichtungen auf einen Finger oder ein Ohrläppchen aufzubringen.

Aus der EP-A2-135 840 ist eine Meßvorrichtung bekannt, mittels derer der Sauerstoffgehalt des kindlichen Blutes dadurch gemessen werden kann, daß ein Sensorträger vaginal eingeführt wird, an dessen freiem Ende eine an einem Kind anbringbare Sensoreinrichtung gelagert ist. Die Messung erfolgt mittels einer Durchleuchtung des kindlichen Gewebes, wobei die Sensoreinrichtung auf die Körperoberfläche des Kindes, beispielsweise im Kopfbereich, aufgelegt wird. Bei dieser Meßvorrichtung erweist es sich als nachteilig, daß eine optische Durchleuchtung des Gewebes dadurch erfolgt, daß der Lichtemitter Licht auf die Oberfläche des kindlichen Gewebes abstrahlt. Da der Lichtempfänger im wesentlichen neben dem Lichtemitter angeordnet ist, also auch außerhalb des Gewebes, erfolgt im wesentlichen keine echte Durchleuchtung oder Transmission des Gewebes.

Aus der GB-A-2 155 618 ist eine Meßvorrichtung bekannt, welche ebenfalls lediglich auf die Oberfläche des kindlichen Körpers aufgebracht wird, ebenso wie die aus der EP-Al-247 777 bekannte Meßvorrichtung.

Der Erfindung liegt die Aufgabe zugrunde, ein Verfahren und eine Vorrichtung der eingangs genannten Art zu schaffen, welche bei einfachem Aufbau und betriebsicherer Durchführbarkeit eine exakte Meßwertaufnahme bei biologischem Gewebe ermöglichen.

Die Aufgabe wird durch die Merkmals Kombinationen der Ausprüche 1 bzw. 2 gelöst.

Die erfindungsgemäße Vorrichtung und das erfindungsgemäße Verfahren zeichnen sich durch eine Reihe erheblicher Vorteile aus. Da erfindungsgemäß zumindest ein Sensorbereich invasiv in das Gewebe eingebracht wird, ist sichergestellt, daß das Gewebe in ausreichender Weise so durchstrahlt wird, daß eine Absorption der Strahlung in Abhängigkeit der zu messenden Parameter erfolgt. Durch die Messung wird das Gewebe nur in minimaler Weise beeinträchtigt und kann beispielsweise ungestört durchblutet werden. Erfindungsgemäß ist es somit möglich, beispielsweise die Sauerstoffsättigung oder die Sauerstoffversorgung des Gewebes auf besonders exakte Weise zu ermitteln oder zu überprüfen. Die Erfindung eignet sich beispielsweise, wie nachfolgend beschrieben werden wird, zur Überwachung eines Kindes während des Geburtsvorganges, es ist jedoch auch möglich, die Sauerstoffversorgung von Experimentaltumoren in der Krebsforschung, die Sauerstoffversorgung des Herzens in der Infarkt-Forschung oder die Überwachung der Sauerstoff-Versorgung bei einem unter besonderen Umgebungsbedingungen befindlichen Menschen zu überwachen, beispielsweise bei Hochleistungssportlern oder bei Arbeitern, welche sich in Räumen befinden, in denen, bedingt durch die geringe Sauerstoffversorgung, eine Erstickungsgefahr droht. Im letzteren Anwendungsfall kann erfindungsgemäß eine Signal- bzw. Warneinrichtung mit der Vorrichtung gekoppelt werden.

Mittels des Verfahrens und der Vorrichtung ist es weiterhin auf besonders einfache Weise möglich, die Durchblutung von Organen und Hautlappen zu überwachen, beispielsweise intra- oder postoperativ, beispielsweise nach Transplantationen, Verschiebeplastiken, Gefäß- und Bypass-Operationen. Weiterhin ist, wie bereits erwähnt, eine perinatale Überwachung möglich.

Da zumindest ein Sensorbereich invasiv eingebracht wird, besteht eine Ausgestaltung der Erfindung darin, daß entweder der strahlungsemittierende oder der strahlungsempfangende Sensorbereich alleine oder aber beide Sensorbereiche invasiv eingebracht werden können.

Bevorzugterweise wird bei dem Verfahren der pulsatile Anteil der Absorption als Meßwert verwendet, da bei diesem pulsatilen Anteil auf besonders einfache Weise die durchblutungsbedingten Einflüsse feststellbar sind.

In einer besonders günstigen Ausgestaltung der Erfindung ist vorgesehen, daß die zur Durchstrahlung des Gewebes verwendete Strahlung aus einem Wellenlängenbereich von 0,1-20 µm ausgewählt wird.

In einer weiteren vorteilhaften Ausgestaltung der Erfindung ist vorgesehen, daß die Wellenlängen der Strahlung (mindestens zwei) zur Ermittlung von Hämoglobinfraktionen so ausgewählt werden, daß es beispielsweise möglich ist, Werte für sHb, sHbO₂` sHbC0₂, sHbCO, sHbS, sMet-Hb, sHbF etc. abzuleiten.

Zur Anbringung der Sensoreinrichtung an dem Gewebe kann es sich als günstig erweisen, wenn diese an einem länglichen Sensorträger, welcher beispielsweise vaginal eingeführt werden kann, angebracht ist.

Um eine betriebssichere, insbesondere durch elektrische Störfelder nicht beeinflußbare Zu- und Ableitung der Strahlung zu gewährleisten, kann es sich als besonders günstig erweisen, wenn die Zu- und/oder Ableitung der Strahlung mittels eines Lichtleiters erfolgt. Dabei kann die Strahlungsquelle an der Sensoreinrichtung oder an einem zugehörigen Meßgerät angeordnet sein.

Erfindungsgemäß ist es auch möglich, die Strahlungsquelle(n) direkt an der Oberfläche des Gewebes anzuordnen, so daß zumindest bei einem Sensorbereich kein Lichtleiter erforderlich ist. Die Erfassung der transmittierten Strahlung kann mittels eines elektro-optischen Wandlers im Meßgerät bei Verwendung eines Lichtleiters, oder direkt an der Oberfläche des Gewebes erfolgen. Es ist auch möglich, elektrooptische Wandler jeweils invasiv in das Gewebe einzuführen. In einer besonders günstigen Ausgestaltung der Erfindung kann der Strahlungssensor und der Strahlungsemitter an einem einstückig ausgebildeten Sensorträger, beispielsweise an einer Nadel angeordnet sein. Es ist jedoch auch möglich, den Strahlungsemitter bzw. den Strahlungssensor jeweils an einem separaten Einstichträger zu lagern. Die Art und Auswahl der jeweiligen Lichtquellen bzw. Lichtsensoren kann auf besonders einfache Weise den jeweiligen Anforderungen angepaßt werden, so ist es beispielsweise möglich, Glühlampen, Leuchtdioden oder Laser(dioden) etc. zur Beleuchtung zu verwenden.

Da der Sensorträger vaginal einführbar ist, ist es möglich, bereits in einem sehr frühen Stadium des Geburtsvorganges die Sensoreinrichtung anzubringen, so daß über einen langen Zeitraum eine Überwachung der Sauerstoffversorgung des Kindes möglich ist, aufgrund derer mögliche therapeutische Schritte, wie etwa die Indikation zum Kaiserschnitt gestellt oder sonstige Entbindungsmodalitäten festgelegt bzw. eingeleitet werden können. Ein weiterer wesentlicher Vorteil der Vorrichtung liegt darin, daß durch die optische Durchleuchtung eines Gewebebereichs des Kindes aussagefähige Meßwerte hinsichtlich der Sauerstoffversorgung des Kindes möglich sind. Da die Sauerstoffversorgung die am schnellsten veränderliche Meßgröße bildet, welche verschiedene Faktoren, wie etwa plazentare Störungen, Störungen der Nabelschnur, Uteruskontraktionen sowie kindliche Notlagen widerspiegelt, ist es über die Messung der fetalen Sauerstoffversorgung möglich, jeweils eine direkte, aktuelle Aussage über den Gesamtzustand des Kindes zu treffen. Die Amplitude des pulsatilen Signals bzw. ihr Integral über die Zeit gibt darüberhinaus Hinweise auf die Durchblutung und korreliert in klinisch aussagefähiger Weise mit einer Zentralisation des fetalen Kreislaufs, d.h. mit einem eventuellen Schockzustand des Feten.

Ein weiterer Vorteil der Vorrichtung ist dadurch gegeben, daß die Durchleuchtung eines Gewebebereiches des Kindes einem physikalischen Prinzip folgt, welches sehr wenig störungsanfällig ist und schnell reagiert, so daß bei einer unzureichenden Anbringung der Sensoreinrichtung ein Fehler sofort feststellbar ist.

Ein weiterer wesentlicher Vorteil der Erfindung liegt in dem Umstand, daß die Vorrichtung über einen langen Zeitraum am Kind belassen werden kann, da das kindliche Gewebe während der Messung weder belastet noch beschädigt wird. Es ist somit nicht notwendig, die Vorrichtung nach einer bestimmten Zeitdauer zu versetzen oder neu anzubringen. Dadurch entfallen nicht nur umständliche und zeitaufwendige Anbringungsvorgänge, es ist auch möglich, den gesamten Geburtsvorgang nach einer einmaligen Justierung oder Einstellung der gesamten Vorrichtung zu überwachen.

Eine besonders günstige Weiterentwicklung der Erfindung ist dadurch gegeben, daß der die Strahlung aussendende Sensorbereich zumindest zwei unterschiedliche, vom Empfänger zu empfangende Wellenlängen abstrahlt. Es ist somit möglich, eine Messung mit einer Wellenlänge durchzuführen, mit welcher der Gesamthämoglobingehalt des Blutes ermittelt werden kann (isosbestischer Punkt), während die zweite Messung bei einer Wellenlänge erfolgt, die dem Oxyhämoglobin angepaßt ist. Da die Transmission im Infrarotbereich vom Hämoglobingehalt in dem durchstrahlten Gewebebereich abhängt, da aber auch andere Faktoren, wie etwa die Hautdicke und ähnliches die Transmission beeinflussen, ist es auf diese Weise möglich, nicht die absoluten Transmissions- oder Durchdringungswerte des durchstrahlten Gewebes festzustellen, sondern nur den durchblutungsbedingten Transmissionswert zu ermitteln.

Bei Beginn der Messung erfolgt ein Abgleich des Meßgerätes, um die konstanten, nicht pulsatilen Signalanteile auszufiltern, beispielsweise in Art einer Wechselsignalkopplung. Es ist somit möglich, in präziser Weise die Sauerstoffsättigung des Blutes zu ermitteln.

In günstiger Weise sind im Bereich der Sensoreinrichtung zumindest eine Lichtquelle und zumindest ein lichtempfindliches Element angeordnet. Bei derartiger Ausgestaltung der Meßvorrichtung ist es möglich, die Sensoreinrichtung mittels elektrischer Anschlüsse mit dem Meßgerät zu verbinden und die elektrische Energie erst unmittelbar im Bereich der Sensoreinrichtung in optische Signale umzuwandeln bzw. die optischen Signale in elektrische Signale rückzuverwandeln. Unter Verwendung miniaturisierter Photohalbleiter ist es möglich, die Sensoreinrichtung in hohem Maße zu miniaturisieren.

Eine andere, bei verschiedensten Anwendungszwecken besonders günstige Ausgestaltung der Vorrichtung ist dadurch gegeben, daß die Sensorbereiche mittels Lichtleitern mit einer am Endbereich des Sensorträgers angeordneten Strahlungsquelle bzw. einem strahlungsempfindlichen Element verbunden sind. Durch die Verwendung von Lichtleitern niedrigen Querschnitts läßt sich zum einen die Dimensionierung der Sensoreinrichtung und des Sensorträgers verringern, zum anderen ist es möglich, größere und/oder leistungsfähigere Photosensoren oder lichtemittierende Elemente zu verwenden. Ein weiterer Vorteil der Verwendung von Lichtleitern ist dadurch gegeben, daß elektromagnetische Störungen die Datenübermittlung von den Sensorbereichen nicht beeinflussen können. Dies führt zu einer erheblichen Steigerung der Meßsicherheit. Weiterhin handelt es sich zumindest bei dem Sensorträger und der mit den Sensorbereichen versehenen Sensoreinrichtung um einmal zu verwendende Artikel. Um im Krankenhausbereich kostengünstig arbeiten zu können, ist es erforderlich, derartige Einwegartikel so kostengünstig als möglich zu produzieren. Dabei erweisen sich Lichtleiter wegen ihrer geringen Herstellungskosten als besonders vorteilhaft. Ein weiterer Vorteil von Lichtleitern liegt darin, daß sie sehr flexibel sind, und so einer Bewegung der Sensoreinrichtung bzw. des Sensorträgers nicht hinderlich sind. Weiterhin ist es möglich, die Lichtleiter mit einem sehr geringen Querschnitt auszubilden, wodurch ein besonders hoher Miniaturisierungsgrad erzielt werden kann. Ein weiterer, sehr wesentlicher Vorteil der Verwendung von Lichtleitern liegt in ihrer Betriebssicherheit, da sie keine gefährlichen elektrischen Ströme leiten können.

Zur sicheren Anbringung der Sensoreinrichtung am Gewebe oder am kindlichen Körper erweist es sich als besonders vorteilhaft, an der Sensoreinrichtung eine Doppelspirale vorzusehen, welche in das Gewebe des Kindes einschraubbar ist und an deren Endbereichen die Sensorbereiche angeordnet sind. Dabei kann es sich als vorteilhaft erweisen, den Sensorträger in Form einer flexiblen Welle auszugestalten, um das Einschrauben der Doppelspirale zu erleichtern.

Eine weitere, ebenfalls sehr günstige Weiterbildung der Vorrichtung ist dadurch gegeben, daß an der Sensoreinrichtung eine mit einem Sensorbereich versehene, in das Gewebe des Kindes schraubbare Spirale vorgesehen ist. Durch diese korkenzieherartige Ausgestaltung ist ein sicheres Einschrauben und Verankern gewährleistet. Auch hierbei kann es sich als vorteilhaft erweisen, den Sensorträger in Form einer flexiblen Welle auszubilden. Der andere Sensorbereich kann in Form eines invasiven Stifts ausgestaltet sein.

Die Ausgestaltung der Spirale bzw. des Stiftes erfolgt in vorteilhafter Weise dadurch, daß diese als röhrenförmiger Hohlkörper ähnlich einer Spritzenkanüle ausgebildet sind, welche jeweils einen oder mehrere Lichtleiter umschließen. Es ist somit möglich, der Spirale bzw. dem Stift eine ausreichende Festigkeit zu verleihen und gleichzeitig sicherzustellen, daß an dem jeweiligen Endbereich der Spirale bzw. des Stiftes ein Strahlungsaustritt bzw. Strahlungseintritt erfolgen kann.

Eine andere Möglichkeit der Ausgestaltung der Sensoreinrichtung, welche ebenfalls besonders vorteilhaft ist, ist dadurch gegeben, daß die Sensorbereiche an der Sensoreinrichtung vorgesehen sind und daß die Sensoreinrichtung eine mit dem Gewebe in Eingriff bringbare zusätzliche Verankerungseinrichtung umfaßt. Die Verankerungseinrichtung kann dabei ebenfalls in Form einer einschraubbaren Spirale oder in Form einer in das Gewebe einstechbaren Nadel ausgebildet sein. Dabei erweist es sich als vorteilhaft, daß die Verankerungseinrichtung nicht zu dem Meßvorgang beiträgt und somit in optimaler Weise dem jeweiligen Verwendungszweck angepaßt werden kann.

Zur Befestigung der Sensoreinrichtung kann in einer weiteren vorteilhaften Ausgestaltung der Erfindung vorgesehen sein, die Sensorbereiche an einer Klammer anzuordnen, mittels derer das Gewebe greifbar ist. Bei dieser Ausgestaltung ist es möglich, Verletzungen der Haut oder des Gewebes durch Einbringen eines Sensorbereichs zu vermeiden. Mittels dieser beschriebenen greifenden Klammer kann es beispielsweise möglich sein, Gliedmaßen, z. B. einen Fußbereich oder einen Fingerbereich, zu ergreifen. Alternativ dazu kann die Klammer auch an ihren vorderen Enden angespitzt sein, so daß es möglich ist, diese in das Gewebe des Kindes einzustechen, um in ähnlicher Weise wie bei den zuvor beschriebenen Sensoreinrichtungen eine Durchstrahlung eines ungestört durchbluteten Gewebebereiches zu ermöglichen.

Weiterhin kann es vorteilhaft sein, die Sensoreinrichtung und damit die Sensorbereiche an dem Körper oder Gewebe anzukleben oder mittels Unterdrucks anzusaugen.

Eine weitere vorteilhafte Ausgestaltung ist dadurch gegeben, daß der Sensorträger flexibel ausgebildet und in einem vorgeformten Führungsrohr angeordnet ist. Das Führungsrohr kann dabei so gekrümmt oder vorgeformt sein, daß das Einführen der Vorrichtung in den weiblichen Körper vereinfacht wird, wodurch auch die Zielgenauigkeit erhöht werden kann.

Bei einer weiteren, sehr vorteilhaften Ausgestaltung sind die Sensoreinrichtung und der Sensorträger mechanisch, nicht jedoch hinsichtlich der Meßwertübertragung entkoppelbar. Dies erweist sich beispielsweise dann als vorteilhaft, wenn die Sensoreinrichtung mittels einer Verankerungseinrichtung in das Gewebe eingeschraubt oder eingesteckt wurde. Durch die mechanische Entkoppelung wird verhindert, daß diese Verankerung unbeabsichtigterweise gelöst wird. Die mechanische Entkoppelung kann dabei durch Ausrücken formschlüssig in Eingriff befindlicher Segmente, beispielsweise durch Zurückziehen des Sensorträgers, bewirkt werden.

Eine weitere, bei Verwendung der Vorrichtung an einem Kind besonders günstige Ausgestaltung ist dadurch gegeben, daß zusätzlich eine Sensoreinrichtung zur Ermittlung der Wehenstärke der Mutter mit dem Meßgerät verbunden ist. Dabei kann es sich um eine übliche Tokografie-Einrichtung handeln. In vorteilhafter Weise weist das Meßgerät eine Anzeigeeinrichtung auf, bei welcher sowohl die Wehenstärke als auch der jeweilige Sauerstoffgehalt des kindlichen Blutes angezeigt wird. Auch ist es möglich, anstelle der Wehenstärke oder zusätzlich ein EKG des Kindes aufzuzeigen.

Im folgenden wird die Erfindung anhand von Ausführungsbeispielen in Verbindung mit der Zeichnung beschrieben. Dabei zeigt:
- Fig. 1: eine schematische Darstellung der erfindungsgemäßen Meßvorrichtung;
- Fig. 2 und 3: schematische Darstellungen von Ausführungsbeispielen der erfindungsgemäßen Sensoreinrichtung;
- Fig. 4: eine Teilansicht eines Ausführungsbeispiels eines Sensorbereichs;
- Fig. 5: ein weiteres Ausführungsbeispiel einer erfindungsgemäßen Sensoreinrichtung;
- Fig. 6: eine Schnittansicht eines weiteren Ausführungsbeispiels der erfindungsgemäßen Sensoreinrichtung, und
- Fig. 7: eine Schnittansicht eines weiteren Ausführungsbeispiels.

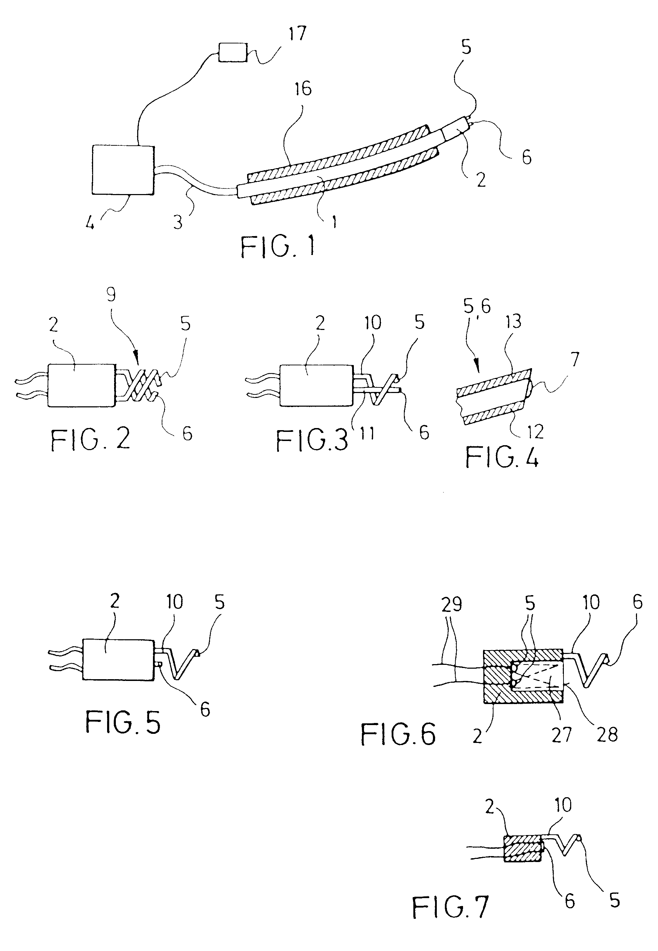

In Fig. 1 ist ein erstes Ausführungsbeispiel der erfindungsgemäßen Vorrichtung in schematischer Darstellung gezeigt. Die Meßvorrichtung umfaßt ein Meßgerät 4, welches zur Vereinfachung der Abbildung nur schematisch als Blackbox dargestellt ist. Mittels Anschlußeinrichtungen 3 ist eine an einem länglichen Sensorträger 1 gelagerte Sensoreinrichtung 2 mit dem Meßgerät 4 verbunden. Der Sensorträger 1 ist in seiner Länge so ausgebildet, daß er transvaginal eingeführt und und die Sensoreinrichtung mit dem kindlichen Körper in Kontakt gebracht werden kann. An dem freien Ende der Sensoreinrichtung 2 sind Sensorbereiche 5, 6 angeordnet, welche im einzelnen nachfolgend noch näher beschrieben werden. Weiterhin ist erfindungsgemäß die Möglichkeit vorgesehen, eine Sensoreinrichtung 17 zur Bestimmung der Wehenstärke (Tokografie) an dem Meßgerät 4 anzuschließen, um nicht nur eine Aussage über den Sauerstoffgehalt des kindlichen Blutes, sondern auch über die Wehentätigkeit der Mutter zu ermöglichen. Alternativ zu der Anzeige der Wehentätigkeit oder zusätzlich zu dieser ist es auch möglich, ein EKG des Kindes aufzunehmen, wobei die am Kind zu befestigende Elektrode einstückig mit einem der Sensorbereiche 5 oder 6 ausgebildet sein kann. In üblicher Weise können die anderen Elektroden zur Aufnahme des EKGs am Körper der Mutter befestigt werden.

Der Sensorträger 1 weist üblicherweise einen möglichst geringen Durchmesser auf, um bereits bei einer geringen Öffnung des Muttermundes einführbar zu sein. Die Sensoreinrichtung 2 ist in bevorzugter Weise mit dem gleichen Außendurchmesser versehen, wie der Sensorträger 1.

Bei Verwendung mancher der nachfolgend beschriebenen Ausführungsbeispiele von erfindungsgemäßen Sensoreinrichtungen kann es vorteilhaft sein, den Sensorträger 1 in Form einer flexiblen Welle auszubilden, um eine Drehung der Sensoreinrichtung 2 zu ermöglichen. Um dabei eine ausreichende Stabilität zu gewährleisten und um zum anderen die Anbringung der Sensoreinrichtung 2 zu erleichtern, kann der Sensorträger 1 in einem Führungsrohr 16 angeordnet sein, welches vorgeformt ist, um den anatomischen Gegebenheiten angepaßt zu sein.

In Fig. 2 ist ein erstes Ausführungsbeispiel der erfindungsgemäßen Sensoreinrichtung 2 dargestellt. An dem freien Ende weist die Sensoreinrichtung eine Doppelspirale 9 auf, an deren Enden jeweils die Sensorbereiche 5, 6 vorgesehen sind. Bei dem Ausführungsbeispiel sendet der Sensorbereich 5 Licht aus, welches von dem Sensorbereich 6 empfangen werden kann. Dabei erweist es sich als vorteilhaft, wenn der Sender 5 zwei verschiedene Lichtwellenlängen abstrahlt, z.B. eine Wellenlänge, welche auf den Gesamthämoglobingehalt abgestimmt ist, sowie eine Wellenlänge, die dem Oxyhämoglobin angepaßt ist. Die Sensoreinrichtung 2 wird auf die kindliche Haut aufgesetzt und gedreht, wodurch die Doppelspirale 9 in das Gewebe des Kindes eingeschraubt wird. Auf diese Weise ist ein Gewebebereich zwischen dem Sender 5 und dem Empfänger 6 eingeschlossen. Das aus dem Sender 5 austretende Licht gelangt somit zum Teil zu dem Empfänger 6, ein Teil des Lichtes wird von dem kindlichen Gewebe absorbiert. Wie bereits beschrieben, ermöglichen die Absorptionsverhältnisse eine Aussage hinsichtlich des Hämoglobingehaltes.

Die Fig. 3 zeigt ein weiteres Ausführungsbeispiel der erfindungsgemäßen Sensoreinrichtung, bei welcher lediglich eine Spirale 10 vorgesehen ist, zu welcher zentrisch ein Stift 11 angeordnet ist. An den freien Enden des Stiftes 11 bzw. der Spirale 10 sind die Sensorbereiche 5 bzw. 6 vorgesehen. Bei dem gezeigten Ausführungsbeispiel ist der Sender 5 am Endbereich der Spirale angeordnet, während der Endbereich des Stiftes 11 den Empfänger 6 aufweist. Die Anordnung kann jedoch auch umgekehrt ausgebildet sein. Bei diesem Ausführungsbeispiel der Sensoreinrichtung wird der Stift 11 bei einer Drehung der Sensoreinrichtung 2 und bei einem Einschrauben der Spirale 10 in das Gewebe des Kindes eingedrückt.

In Fig. 4 ist in vergrößerter Darstellung, im Schnitt, ein Endbereich einer der in den Fig. 2 oder 3 gezeigten Spiralen oder des Stiftes 11 dargestellt. Diese sind, wie aus Fig. 4 ersichtlich ist, in Form eines Hohlkörpers 13, beispielsweise eines Rohres, ausgebildet, in dessen Innenraum ein Lichtleiter 12 angeordnet ist. Am Ende des Hohlkörpers 13 befindet sich somit eine nur schematisch dargestellte Lichtquelle 7 bzw. das Ende eines Lichtleiters zur Aussendung von Licht. In ähnlicher Weise ist es möglich, den Empfänger 6 auszubilden, wobei anstelle der Lichtquelle 7 ein lichtempfindliches Element 8 bzw. das Ende eines Lichtleiters (siehe Fig. 8) vorgesehen sein kann.

Das in Fig. 5 gezeigte Ausführungsbeispiel unterscheidet sich von dem Ausführungsbeispiel gemäß Fig. 3 darin, daß nur ein Sensorbereich, nämlich der Sensorbereich 5, invasiv in das Gewebe einführbar ist, während der andere Sensorbereich 6 bei invasiv eingeschraubtem Sensorbereich 5 gegen die Oberfläche des Gewebes anliegt. Die Anordnung kann jedoch auch umgekehrt sein. Es ist somit sichergestellt, daß zum einen eine exakte Verankerung der Sensoreinrichtung 2 erfolgen kann, andererseits nur ein Sensorbereich invasiv eingebracht werden muß.

In Fig. 6 ist in der Schnittansicht ein weiteres Ausführungsbeispiel dargestellt, welches in seinem grundsätzlichen Aufbau dem Ausführungsbeispiel von Fig. 5 ähnelt. Die Sensoreinrichtung 2 weist an ihrem vorderen Ende eine Spirale 10 auf, an deren Ende ein Empfänger 6 angeordnet ist. Im Inneren der Sensoreinrichtung 2 ist ein Strahlungstransparenter Körper 27 vorgesehen, welcher eine strahlungsdurchlässige Fläche 28 aufweist, die beispielsweise an die Haut eines Kindes anlegbar und an dem kindlichen Gewebe mittels der Spirale 10 verankerbar ist. Anstelle des Körpers 27 kann auch ein Hohlraum vorgesehen sein. An dem bodenseitigen Bereich des Körpers 27 sind zwei Sensorbereiche 5 angeordnet, welche in Form von Strahlungsemittern ausgebildet sind. Diese sind über Anschlüsse 29, welche beispielsweise in Form von Lichtleitern oder elektrischen Anschlüssen ausgestaltet sein können, verbunden. Die beiden Strahlungsemitter 5 geben jeweils eine Strahlung unterschiedlicher Wellenlänge ab, welche sich in dem Körper 27, wie gestrichelt dargestellt, kegelartig ausbreitet. Dies führt zu einer Mischung der beiden Wellenlängen am Bereich der Austritts- oder Kontaktfläche 28 und stellt sicher, daß der Austritt der Strahlung und die Einleitung der Strahlung in das Gewebe über eine relativ große Fläche erfolgt. Eine teilweise Abdeckung oder Behinderung des Strahlenganges durch im Gewebe befindliche Hindernisse, beispielsweise Blutgefäße, führt nicht zu einer Beeinträchtigung der Durchstrahlung und ermöglicht eine ausreichende Zuleitung der Strahlen zu dem Empfänger 6.

In Fig. 7 ist die Schnittansicht eines weiteren Ausführungsbeispiels dargestellt, das ebenfalls in seinem grundsätzlichen Aufbau dem Ausführungsbeispiel von Fig. 5 ähnelt. Die Sensoreinrichtung 2 weist an ihrem vorderen Ende eine Spirale 10 auf, an deren Verlauf oder Ende ein Sender 5 angebracht ist. Am vorderen Ende der Sensoreirichtung 2 ist ein Strahlungsempfänger 6 angebracht. Der besondere Vorteil dieses Ausführungsbeispiels besteht darin, daß der Strahlungsempfänger besonders großflächig ausgeführt werden kann.

Die Erfindung ist nicht auf die gezeigten Ausführungsbeispiele beschränkt, vielmehr ist es im Rahmen der Erfindung möglich, weitere Ausgestaltungsformen der Sensoreinrichtung auszubilden. Erfindungsgemäß ist es lediglich erforderlich, ein strahlungsaussendendes Element vorzusehen, welches einen Gewebebereich durchstrahlen kann, wobei die Strahlung von einem Empfänger oder strahlungsempfindlichen Element aufgefangen wird, um den Absorptionsgrad ermitteln zu können. Erfindungsgemäß ist es somit möglich, biologisches Gewebe beliebiger Art zu untersuchen, nicht nur zur Überwachung des Geburtsvorganges, sondern auch im Rahmen der Forschung oder zur kontinuierlichen Überwachung. So ist es erfindungsgemäß möglich, zur Überwachung, beispielsweise des Geburtsvorganges, die in oben beschriebener Weise ermittelten Werte der Sauerstoffsättigung mit der Wehentätigkeit, durch eine Tokographieeinrichtung ermittelt werden kann, und/oder der kindlichen Herzfrequenz, die durch ein fetales EKG oder durch Ultraschall ermittelt werden kann, zu korrelieren, um auf diese Weise Artefakte zu unterdrücken.

## Patentansprüche

1. Verfshren zur Messung der Absorption von Strahlung in einem Gewebe mittels einer Sensoreinrichtung (2), welche zumindest einen (5) als Strahlungsemitter und zumindest einen (6) als Strahlungsempfänger ausgebildeten Sensorbereich umfabt, dadurch gekennzeichnet, daß zumindest ein Sensorbereich zur Durchstrahlung des Gewebes invasiv in das Gewebe gestochen wird.

2. Vorrichtung zur Messung der Absorption von Strahlung in einem Gewebe mit einer Sensoreinrichtung (2), welche zwei mit einem Meßgerät (4) betriebsverbundene Sensorbereiche (5,6) zur Durchstrahlung eines Gewebebereiches umfaßt, wobei ein Sensorbereich (5) als Strahlungsemitter und der andere Sensorbereich (6) als Empfänger ausgebildet ist, dadurch gekennzeichnet, daß zumindest ein Sensorbereich (5,6) zur Durchstrahlung des Gewebebereiches invasiv in das Gewebe einstechbar ausgebildet ist.

3. Vorrichtung nach Anspruch 2, dadurch gekennzeichnet, daß die Wellenlängen der Strahlung aus einem Wellenlängenbereich von 0,1 - 20 µm ausgewählt sind.

4. Vorrichtung nach einem der Ansprüche 2 oder 3, dadurch gekennzeichnet, daß die Wellenlängen der Strahlung zur Ermittlung von Hämoglobin-Fraktionen ausgewählt sind.

5. Vorrichtung nach einem der Ansprüche 2-4, dadurch gekennzeichnet, daß die Sensoreinrichtung (2) an einem Sensorträger (1) angebracht ist.

6. Vorrichtung nach einem der Ansprüche 2-5, dadurch gekennzeichnet, daß die Zu- und/oder Ableitung der Strahlung mittels eines Lichtleiters (12) erfolgt.

7. Vorrichtung nach einem der Ansprüche 2-6, dadurch gekennzeichnet, daß der die Strahlung aussendende Sensorbereich (5) zumindest zwei unterschiedliche vom Empfänger zu empfangende Wellenlängen abstrahlt.

8. Vorrichtung nach einem der Ansprüche 2-7, dadurch gekennzeichnet, daß an der Sensoreinrichtung (2) eine mit einem Sensorbereich versehene, in das Gewebe schraubbare Spirale angeordnet ist.

9. Vorrichtung nach einem der Ansprüche 2-8, dadurch gekennzeichnet, daß die Spirale als den Lichtleiter (12) umschließender, röhrenförmiger Hohlkörper (13) ausgebildet ist.

10. Vorrichtung nach einem der Ansprüche 2-9, dadurch gekennzeichnet, daß zusätzlich eine Sensoreinrichtung (17) zur Ermittlung weiterer Meßwerte mit dem Meßgerät (4) verbunden ist.

## Claims

1. Method for measuring the absorption of radiation in a portion of tissue by means of a sensor device (2) comprising sensor areas of which at least one is a radiation emitter (5) and at least one is a radiation receiver (6), and wherein at least one sensor area can be invasively introduced into the tissue for transilluminating the same.

2. Device for measuring the absorption of radiation in a portion of tissue by means of a sensor device (2) comprising two sensor areas (5, 6) for transilluminating said tissue and which are connected to a measuring instrument (4), with one sensor area (5) being designed as a radiation emitter and the other (6) being designed as a radiation receiver, and wherein at least one sensor area (5, 6) is suitable for invasive introduction into said tissue for transilluminating the same.

3. Device according to claim 2, wherein the wavelengths of the radiation are selected within a wavelength range of 0.1 - 20 µm.

4. Device according to claim 2 or 3, wherein the wavelengths of the radiation are selected with the aim of determining haemoglobin fractions.

5. Device according to one of the claims 2 to 4, wherein the sensor device (2) is mounted on a sensor carrier (1).

6. Device according to one of the claims 2 to 5, wherein radiation is emitted and/or detected by a light-transmitting fibre (12).

7. Device according to one of the claims 2 to 6, wherein the radiation-emitting sensor area (5) emits radiation of at least two different wavelengths to be received by the receiver.

8. Device according to one of the claims 2 to 7, wherein the sensor device (2) is provided with a spiral carrying a sensor area, and which is suitable for screwing into the tissue.

9. Device according to one of the claims 2 to 8, wherein the spiral consists of a tubular hollow body (13) which houses the light-transmitting fibre (12).

10. Device according to one of the claims 2 to 9, wherein an additional sensor device (17) for determination of further parameters is connected to the measuring instrument (4).

## Revendications

1. Procédé et dispositif pour mesurer l'absorption de rayonnements à l'aide d'un dispositif de capteurs (2) qui comprend au moins un capteur (5) sous la forme d'un capteur émetteur et au moins un capteur (6) sous la forme d'un capteur récepteur qui sont caractérisés par le fait que au moins un des capteurs soit piqués directement dans le tissu afin de permettre la transmission du rayon au travers du tissu.

2. Dispositif servant à mesurer l'absorption de rayonnement par un tissu a l'aide d'une installation de capteurs (2) qui comprend deux capteurs (5) et (6) reliés à un appareil de mesure (4) afin de pouvoir traverser un tissu par un rayon. Pour ce faire, un des capteurs est un émetteur et l'autre est un récepteur, la caractéristique principale étant qu'au moins un des capteurs est introduit directement dans le tissu pour procéder à la transmission du rayonnement au travers de celui-ci.

3. D'après l'exigence 2, le dispositif est caractérisé par le fait que les longueurs d'ondes du rayonnement sont choisis dans un intervalle de longueur d'ondes compris entre 0,1-20 pm.

4. D'après les spécifications 2 ou 3, le dispositif est caractérisé par le fait que les longueurs d'ondes du rayonnement soient choisies afin de donner des renseignement sur les fractions d'hémoglobine.

5. D'après les spécifications 2-4, le dispositif est caractérisé par le fait que l'installation de capteur (2) soit fixée sur un porteur de capteur.

6. D'après une des spécifications 2-5, le dispositif est caractérisé par le fait que l'émission et le captage du rayonnement se fasse à l'aide d'un conducteur de lumière (12).

7. D'après une des spécifications 2-6, le dispositif est caractérisé par le fait que le capteur (5) émetteur du rayonnement émette au moins deux longueurs d'ondes différentes qui seront captées par le récepteur.

8. D'après une des spécifications 2-7, le dispositif est caractérisé par le fait qu'une spirale soit adaptée à l'installation de capteur. Cette spirale est munie d'un capteur et se fixe à la manière d'une vis dans le tissu.

9. D'après une des spécifications 2-8, le dispositif est caractérisé par le fait que la spirale, corps creux en forme de tube qui enserre le conducteur de lumière.

10. D'après une des spécifications 2-9, le dispositif est caractérisé par le fait qu'un autre installation de capteurs soit reliée à l'appareil de mesure afin de donner des indications sur d'autres valeurs.
